# FASCICULE DE BREVET EUROPEEN

(11) **EP 0 766 544 B1**
(45) Date de publication et mention de la délivrance du brevet: **13.05.1998**
(21) Numéro de dépôt: 95923418.8
(22) Date de dépôt: 20.06.1995
(51) Int. Cl.: A61F 9/00

(54) **IMPLANT DE SCLEROTOMIE**
SKLEROTOMIE-IMPLANTAT
SCLEROTOMY IMPLANT

(30) Priorité: 22.06.1994 FR 9407758
(43) Date de publication de la demande: 09.04.1997
(73) Titulaire: Chauvin Opsia, 31320 Labege (FR)
(72) Inventeur: PYNSON, Joel, F-31000 Toulouse (FR); PAYROU, Viviane, F-31400 Toulouse (FR); FEURER, Bernard, F-31450 Montlaur (FR)
(74) Mandataire: Cabinet BARRE LAFORGUE & associés
(86) Numéro de dépôt international: FR9500818
(87) Numéro de publication internationale: WO9535078

(56) Documents cités:
- WO-A-91/07195
- WO-A-92/00112
- FR-A- 2 093 736
- US-A- 4 521 210
- US-A- 5 073 163
- US-A- 5 342 370
- OPHTALMOLOGIE, vol. 2, 1988 pages 227-229, M. KAMOUN ET AL. 'MICROTRABECULOPROTHESE' cité dans la demande
- KOREAN J. OPHTHALMOL., vol. 2, 1988 HYUN BONG BAE ET AL. 'A MEMBRANOUS DRAINAGE IMPLANT IN GLAUCOMA FITERING SURGERY ANIMAL TRIAL' cité dans la demande

## Description

L'invention concerne un implant destiné à être mis en place dans la sclère de l'oeil à l'issue d'une opération de sclérotomie avec ou sans trabéculectomie.

Actuellement le glaucome est soigné dans les cas les plus graves par une intervention chirurgicale désignée trabéculectomie. Cette intervention consiste, après désinsertion conjonctivale, à réaliser une trappe dans la sclère par découpage d'un volet scléral (clivage des plans de la sclère jusqu'au trabéculum) et à inciser le trabéculum pour permettre un écoulement d'humeur aqueuse contenue dans la chambre antérieure, ce qui diminue la tension intraoculaire et limite les conséquences pathologiques du glaucome. Une telle intervention qui est pratiquée depuis une quinzaine d'années environ, engendre généralement une amélioration temporaire, mais à terme, la cicatrisation du volet scléral est susceptible d'obturer l'écoulement d'humeur aqueuse et l'on constate alors assez rapidement une nouvelle augmentation de la pression intra-oculaire.

On a tenté de pallier ce défaut en appliquant des substances antimitotiques sur le volet scléral afin de retarder sa cicatrisation. Toutefois, les résultats sont inconstants et on peut même observer un écoulement excessif d'humeur aqueuse qui peut entraîner une chute trop importante de la pression intra-oculaire.

Un autre type d'intervention est actuellement pratiqué pour soigner le glaucome : elle consiste à mettre en place une valve associée à un conduit qui débouche dans la chambre antérieure pour permettre un écoulement d'humeur aqueuse en cas d'élévation de pression dans cette chambre. Cette technique présente toutefois des inconvénients : en premier lieu, elle exige une opération complexe affectant la chambre antérieure, avec risque délétère sur l'endothélium cornéen (les chirurgiens habitués aux trabéculectomies sont souvent réticents à pratiquer ce type d'intervention chirurgicale complexe et risquée). En outre, le conduit a tendance à se boucher de sorte que l'efficacité du dispositif qui est bonne après mise en place, décroît rapidement dans le temps. De plus, le conduit est parfois expulsé de la chambre antérieure. Il est à noter que ces valves ont une structure relativement complexe qui en rend la fabrication onéreuse.

Par ailleurs, plusieurs publications ont proposé de nouvelles techniques qui en sont au stade expérimental (ou en sont restées au stade expérimental en raison de défauts rédhibitoires) et qui consistent à insérer un implant sous le volet scléral en vue d'éviter l'obturation trop rapide de l'écoulement d'humeur aqueuse à travers l'incision du trabéculum.

Une première technique est décrite dans la publication suivante : "Hyun Bong Bae et al, A Membranous Drainage Implant in Glaucoma Filtering Surgery : Animal Trial, Kor. J. Ophtalmol., vol. 2, 1988, 49-56". Elle consiste à disposer sous le volet scléral une membrane hydrophobe qui pénètre dans la chambre antérieure par l'incision du trabéculum. Grâce à son caractère hydrophobe, cette membrane s'oppose à la formation de fibroses à proximité de sa surface et permet un écoulement de l'humeur aqueuse le long de celle-ci. Toutefois, l'écoulement permanent ainsi conditionné est faible et la présence d'une telle membrane paraît insuffisante pour ramener la pression intra-oculaire à une valeur normale. De plus, sa mise en place implique une intervention au niveau de la chambre antérieure avec des risques pour l'endothélium cornéen, cette intervention étant beaucoup plus délicate qu'une simple trabéculectomie comme déjà indiqué.

La publication suivante : "M. Kamoun et al, Microtrabéculoprothèse, Ophtalmologie, 1988, vol. 2, 227-229", décrit une trabéculoprothèse réalisée en un hydrogel poreux très hydrophile qui est mis en place à sec dans la sclère de façon à pénétrer dans l'incision du trabéculum par une partie effilée ; l'hydratation engendre un gonflement de l'hydrogel et une fermeture hermétique de l'incision : l'humeur aqueuse s'écoule alors à travers les porosités du matériau, depuis la chambre antérieure jusqu'à la sclère. Cette filtration à travers un tel matériau poreux assure une baisse de la pression intraoculaire. Toutefois, le défaut précité résidant dans une obturation rapide de l'écoulement n'est pas supprimé par cette prothèse, car les fibroses qui se forment au niveau de la sclère conduisent rapidement à une obturation de l'écoulement. De plus, comme pour la technique précédente, la mise en place de cette prothèse affecte l'endothélium puisqu'une partie effilée de la prothèse est amenée à pénétrer à l'intérieur de la chambre antérieure.

Le brevet US 4.521.210 décrit un implant de forme allongée qui est disposé au-dessus de la choroïde à l'interface de celle-ci et de la sclère. Cet implant possède une partie effilée qui pénètre dans la chambre antérieure. Il présente les mêmes inconvénients que le précédent, même si sa grande longueur et les trous et passages qui y sont ménagés assurent une meilleure évacuation de l'humeur aqueuse. En outre, sa mise en place paraît extrêmement délicate compte tenu des dimensions de cet implant et de sa position à l'interface choroïde/sclère.

Un autre implant est décrit dans la publication suivante : "Nancy Michel, A wick that promotes fluid drainage treats glaucoma successfully in Russia, Ocular Surgery News 1993, vol. 11 (23) p 26". Cet implant est constitué par un faisceau de fibres résorbables, qui est mis en place sous le volet scléral en regard de l'incision du trabéculum. Les inventeurs ont peu d'informations sur cette technique proposée très récemment. Toutefois, il est probable qu'un tel implant d'origine biologique (porcine), noyé dans la sclère, subisse les mêmes problèmes d'obturation que les implants précédents. En outre, des déplacements dans la trappe, voire une expulsion, sont à craindre.

Un autre implant est décrit dans la demande internationale WO-A-9107195. Cet implant inséré dans la sclère ne résout pas le défaut précité d'obturation de l'écoulement par les fibroses qui se forment dans ce tissu.

Il est à noter que tous les implants connus précédemment évoqués sont posés au terme d'une trabéculectomie au cours de laquelle le trabéculum est incisé.

La présente invention se propose de fournir un nouvel implant remédiant aux défauts précités et présentant un effet de longue durée dans la thérapie du glaucome.

Un premier objectif de l'invention est de fournir un implant permettant un écoulement satisfaisant de l'humeur aqueuse de la chambre antérieure, écoulement continu soumis à des risques réduits d'obturation dans le temps.

Un autre objectif est de fournir un implant susceptible d'être mis en place soit par une simple opération de sclérotomie au cours de laquelle le trabéculum n'est pas touché, soit par une intervention de trabéculectomie n'affectant pas la chambre antérieure de l'oeil et l'endothélium cornéen (cet implant qui n'exige pas nécessairement une trabéculectomie sera désigné par "implant de sclérotomie").

Un autre objectif est de fournir un implant non soumis à des risques de déplacements ou d'expulsion.

Un autre objectif est de fournir un implant de structure simple se prêtant à une fabrication aisée.

A cet effet, l'implant de sclérotomie visé par la présente invention se présente sous la forme d'une plaquette en un matériau biocompatible et est caractérisé en ce qu'il comprend, en combinaison :
- une première extrémité, dite extrémité trabéculaire, adaptée pour pouvoir se disposer contre le trabéculum, qui peut être laissé intact ou subir une trabéculectomie comme dans les techniques traditionnelles,
- une première partie, dite intra-sclérale, portant l'extrémité trabéculaire et adaptée pour pouvoir être insérée dans une trappe sous un volet scléral,
- une seconde partie, dite sous-conjonctivale, prolongeant la partie intra-sclérale et d'épaisseur (e₂) inférieure à celle (e₁) de ladite partie intra-sclérale, ladite partie sous-conjonctivale étant adaptée pour pouvoir sortir de la sclère et se loger sous la conjonctive au contact de celle-ci,
- des moyens d'arrêt situés à la jonction de la partie intra-sclérale et de la partie sous-conjonctivale et adaptés pour pouvoir assurer un blocage de ladite partie intra-sclérale dans la trappe,
- une seconde extrémité, dite extrémité externe, opposée à l'extrémité trabéculaire et portée par la partie sous-conjonctivale,
- des moyens d'écoulement continu s'étendant le long des partie intra-sclérale et partie sous-conjonctivale entre l'extrémité trabéculaire et l'extrémité externe en vue de permettre un écoulement de l'humeur aqueuse depuis le trabéculum vers la conjonctive, soit par filtration à travers le trabéculum si celui-ci est laissé intact, soit par l'incision pratiquée en cas de trabéculectomie.

Selon un premier mode de réalisation, ces moyens d'écoulement continu comprennent au moins une rainure continue pratiquée le long des partie intra-sclérale et partie sous-conjonctivale de façon à déboucher au niveau des extrémité trabéculaire et extrémité externe.

L'implant peut également être réalisé en un matériau poreux, les moyens d'écoulement continu étant constitués par des passages internes provenant de la porosité du matériau.

Selon un mode de réalisation préférentiel, les deux types de moyens d'écoulement sont combinés : le matériau de l'implant est poreux et les moyens d'écoulement comprennent, d'une part, au moins une rainure continue pratiquée le long des partie intra-sclérale et partie sous-conjonctivale, d'autre part, des passages internes provenant de la porosité du matériau.

Selon une autre caractéristique avantageuse de l'invention, l'extrémité trabéculaire présente une forme concave de rayon de courbure sensiblement compris entre 4 mm et 8 mm. Cette extrémité épouse ainsi la forme du trabéculum au contact duquel elle est disposée, ce qui favorise le recueil de l'humeur aqueuse et son évacuation à travers les moyens d'écoulement de l'implant (en particulier en l'absence de trabéculectomie, dans le cas où le trabéculum est laissé intact).

Les moyens d'arrêt situés à la jonction de la partie intra-sclérale et de la partie sous-conjonctivale comprennent de préférence des épaulements, disposés de part et d'autre de la partie intra-sclérale et définis par une largeur de ladite partie intra-sclérale plus grande que celle de la partie sous-conjonctivale. Comme on le verra plus loin, ces épaulements viennent coopérer avec les points de suture qui sont pratiqués à la fin de l'intervention pour maintenir le volet scléral dans la trappe au-dessus de l'implant : la partie intra-sclérale se trouve ainsi bloquée dans une position stable entre le trabéculum et ces points de suture. Dans cette position, la partie sous-conjonctivale qui prolonge ladite partie intra-sclérale, est disposée sous la conjonctive au contact de celle-ci et permet à l'humeur aqueuse circulant dans les moyens d'écoulement de l'implant de sortir de la sclère pour se déverser sous la conjonctive sans risque d'obturation.

D'autres caractéristiques et avantages de l'invention se dégageront de la description qui suit en référence aux dessins annexés, lesquels présentent, à titre non limitatif, deux modes de réalisation d'implants conformes à l'invention et illustrent l'utilisation de ceux-ci pour soigner le glaucome de l'oeil ; sur ces dessins :
- les figures 1, 2, 3 et 4 représentent -à échelle très dilatée (échelle 10 environ)- un implant respectivement en perspective, en vue frontale selon V₁, en coupe AA' et en vue de dessous selon V₂,
- les figures 5, 6, 7 et 8 illustrent schématiquement la mise en place dudit implant au cours d'une trabéculectomie,
- la figure 9 représente en perspective une variante d'implant,
- la figure 10 illustre cet implant posé au terme d'une simple opération de sclérotomie n'affectant pas le trabéculum.

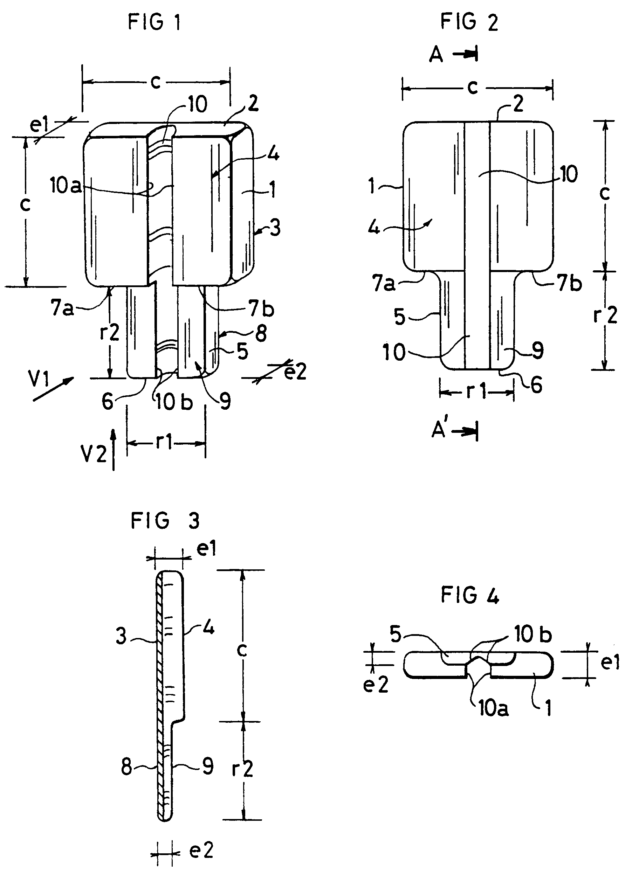

L'implant représenté à titre d'exemple aux figures 1 à 4 se présente sous la forme d'une mince plaquette allongée en un matériau biocompatible, en particulier en copolymère méthylmétacrylate/vinylpyrolidone. Les proportions relatives des deux composés sont notamment d'environ 48 % et 52 %. Un tel copolymère présente une porosité élevée qui peut être mesurée par son taux d'hydrophilie de l'ordre de 40 % (masse d'eau susceptible d'être absorbée rapportée à la masse de copolymère sec). Les dimensions fournies plus loin à titre non limitatif correspondent à l'implant hydraté et diminue d'environ 20 % lorsque l'implant est sec.

L'implant comprend essentiellement deux parties en forme générale de parallélépipède rectangle : une partie intra-sclérale 1 et une partie sous-conjonctivale 5 plus étroite que la première.

La partie intra-sclérale est dotée d'une extrémité trabéculaire 2, en l'exemple de forme plane, qui est appelée à venir se disposer en regard du trabéculum et dont les dimensions sont supérieures à celles de l'incision (qui dans cet exemple sera pratiquée dans celui-ci) de façon à être arrêtée par le trabéculum. Cette extrémité présentera en pratique une largeur -c- sensiblement comprise entre 2 mm et 4 mm, en l'exemple égale à 3 mm (à ± 20 % près) de façon à dépasser de part et d'autre de l'incision du trabéculum (dont la longueur est toujours inférieure à 2 mm).

La partie intra-sclérale 1 possède une face dite supérieure 3 et une face dite inférieure 4 de forme sensiblement carrée de côté -c- égal à 3 mm (à ± 20 % près). L'épaisseur e₁ de ladite partie intra-sclérale est en particulier de 0,50 mm (à ± 20 % près).

La partie sous-conjonctivale 5 qui prolonge la partie intra-sclérale ci-dessus décrite se termine par une extrémité dite externe 6 et présente des dimensions inférieures à celles de la partie intra-sclérale. La jonction entre les deux parties définit deux épaulements 7a, 7b situés de part et d'autre de la partie intra-sclérale ; ces épaulements sont appelés à faire fonction de moyens d'arrêt assurant un blocage de l'implant lors de l'exécution des points de suture en fin d'intervention.

La partie sous-conjonctivale 5 possède une face supérieure 8 et une face inférieure 9 de forme rectangulaire, ayant un côté r₁ égal à 1,5 mm (à ± 20 % près) dans le sens de la largeur et un côté r₂ égal à 2 mm (à ± 20 % près) dans le sens de la longueur.

La partie sous-conjonctivale possède une épaisseur -e₂- qui est en particulier égale à 0,25 mm (à ± 20 % près) (de l'ordre de la moitié de l'épaisseur -e₁-de la partie intra-sclérale).

Comme le montrent les figures, les parties intra-sclérale et sous-conjonctivale possèdent, en l'exemple représenté, des faces inférieures 3 et 8 situées sensiblement dans le même plan et des faces supérieures opposées 4 et 9 qui sont décalées de sorte que la face supérieure de la partie sous-conjonctivale 5 se trouve en retrait par rapport à celle de la partie intra-sclérale 1.

Par ailleurs, une rainure continue 10 est ménagée le long de l'implant dans les parties intersclérale 1 et sous-conjonctivale 5 de façon à déboucher, de part et d'autre de l'implant, au niveau des extrémité trabéculaire 2 et extrémité externe 6. Cette rainure est en l'exemple disposée selon l'axe de l'implant et possède une section dont le fond est en forme de section circulaire ou en angle ouvert ; dans la partie intra-sclérale 1, elle s'ouvre sur la face supérieure 4 par une portion à faces parallèles 10a, cependant qu'elle s'ouvre sur la face supérieure 9 de la partie sous-conjonctivale par des faces divergentes 10b.

La plaquette ci-dessus décrite est utilisée comme implant de sclérotomie (avec ou sans trabéculectomie) pour soigner le glaucome de l'oeil grâce à une implantation dans l'oeil telle que :
. l'extrémité trabéculaire 2 soit disposée contre le trabéculum et arrêtée par ce dernier sans pénétration dans la chambre antérieure,
. la partie sous-conjonctivale 5 soit disposée sous la conjonctive au contact de celle-ci,
. les moyens d'arrêt 7a, 7b coopèrent avec des points de suture S₁, S₂ pour assurer un blocage de la partie sous-conjonctivale 1 dans la trappe T.

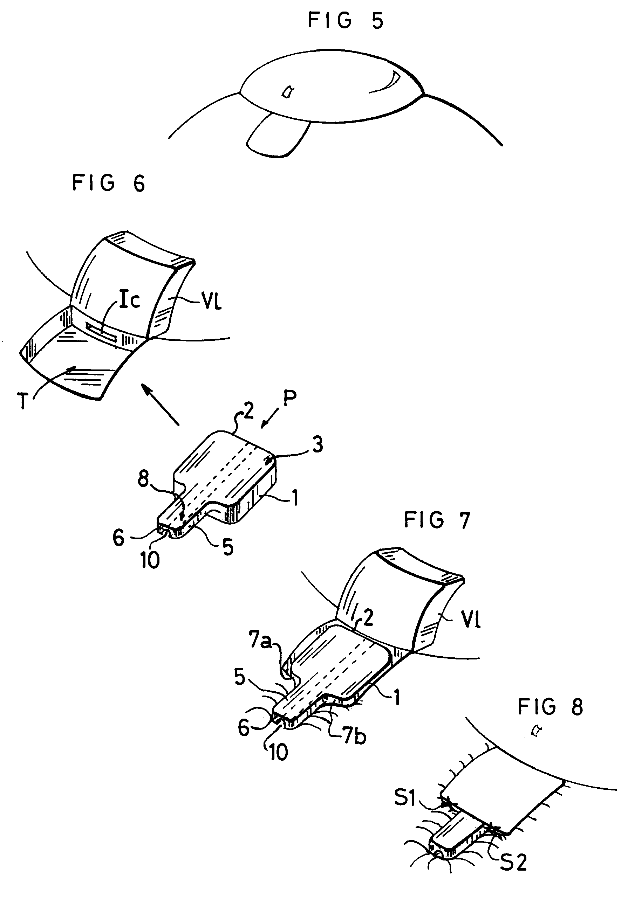

Les figures 5 à 8 illustrent la mise en place dans un oeil dans le cas où est pratiquée une trabéculectomie. L'implant est préalablement imprégné d'une solution aqueuse jusqu'à saturation.

Par une intervention de sclérotomie, après désinsertion conjonctivale, un volet scléral Vₗ est découpé dans la sclère de façon à réaliser une trappe T. Le trabéculum est ensuite incisé. La figure 6 montre cette incision en I_{c}.

L'implant P est présenté comme le montre cette figure avec son extrémité trabéculaire 2 du côté du trabéculum et son extrémité externe 6 du côté de la conjonctive ; la face supérieure 3, 8 est disposée au-dessus et la face inférieure 4, 9 qui est pourvue de la rainure 10 est située en regard des tissus.

Comme l'illustre la figure 7, l'implant est inséré dans l'oeil de façon que sa partie intra-sclérale 1 vienne se loger dans la trappe T avec son extrémité trabéculaire 2 contre le trabéculum en regard de l'incision I_{c}, et que sa partie sous-conjonctivale 5 vienne en appui contre la conjonctive à l'extérieur de la trappe ; dans cette position, l'extrémité externe 6 se trouve à l'extérieur de la sclère contre la conjonctive.

Le volet scléral Vₗ est ensuite rabattu vers la trappe et des points de suture S₁, S₂ sont pratiqués dans le creux des épaulements 7a et 7b de l'implant pour fixer le volet sur la sclère. La partie intra-sclérale de l'implant se trouve alors bloquée dans la trappe entre le trabéculum et ces points de suture. La conjonctive peut ensuite être amenée à recouvrir l'ensemble de façon classique.

La rainure 10 et les porosités de copolymère forment un écoulement continu entre l'incision trabéculaire et la conjonctive sans risque d'obturation quelle que soit la formation de fibroses dans la sclère.

Il convient de souligner que l'intervention chirurgicale ci-dessus résumée est de type traditionnel : elle n'affecte pas la chambre antérieure et ne fait courir aucun risque à l'endothélium cornéen.

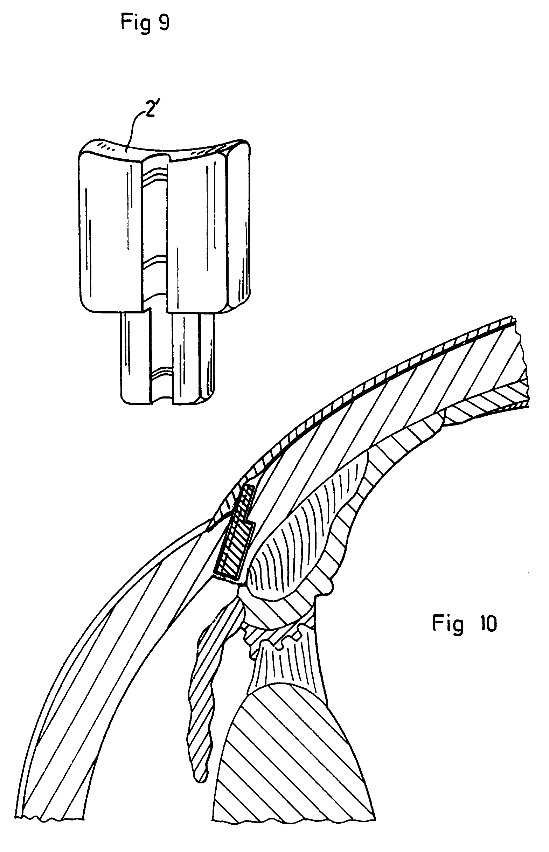

La figure 9 présente une variante d'implant dont les caractéristiques sont identiques au précédent, à l'exception de la forme de l'extrémité trabéculaire référencée 2' à cette figure. Cette extrémité est concave, sensiblement cylindrique, de rayon de courbure sensiblement égal à 6 mm (à ± 20 % près).

L'opération de mise en place est analogue à la précédente à l'exception de l'incision du trabéculum qui n'est pas pratiquée. L'extrémité trabéculaire est disposée au contact du trabéculum de façon à épouser la surface de celui-ci. Le passage d'humeur de la chambre antérieure s'effectue par filtration à travers le trabéculum.

La figure 10 illustre en coupe l'implant posé : il est essentiel de noter que sa partie sous-conjonctivale sort de la sclère et est logée sous la conjonctive. Ainsi, l'extrémité externe déverse l'humeur aqueuse sous la conjonctive et forme une bulle de filtration sous celle-ci. Cette bulle sert de témoin d'efficacité de l'implant : visible à travers la conjonctive, elle permet de vérifier que l'écoulement s'effectue correctement.

## Revendications

1. Implant de sclérotomie destiné à permettre un écoulement continu d'humeur aqueuse à travers le trabéculum d'un sujet, ledit implant se présentant sous la forme d'une plaquette en un matériau biocompatible et étant caractérisé en ce qu'il comprend, en combinaison :
- une première extrémité, dite extrémité trabéculaire (2), adaptée pour pouvoir se disposer contre le trabéculum,
- une première partie, dite intra-sclérale (1), portant l'extrémité trabéculaire (2) et adaptée pour pouvoir être insérée dans une trappe sous un volet scléral,
- une seconde partie, dite sous-conjonctivale (5), prolongeant la partie intra-sclérale (1) et d'épaisseur (e₂) inférieure à celle (e₁) de ladite partie intra-sclérale, ladite partie sous-conjonctivale (5) étant adaptée pour pouvoir sortir de la sclére et se loger sous la conjonctive au contact de celle-ci,
- des moyens d'arrêt (7a, 7b) situés à la jonction de la partie intra-sclérale (1) et de la partie sous-conjonctivale (5) et adaptés pour pouvoir assurer un blocage de ladite partie intra-sclérale dans la trappe,
- une seconde extrémité, dite extrémité externe (6), opposée à l'extrémité trabéculaire (2) et portée par la partie sous-conjonctivale (5),
- des moyens d'écoulement continu (10) s'étendant le long des partie intra-sclérale (1) et partie sous-conjonctivale (5) entre l'extrémité trabéculaire (2) et l'extrémité externe (6) en vue de permettre un écoulement de l'humeur aqueuse depuis le trabéculum vers la conjonctive.

2. Implant selon la revendication 1, caractérisé en ce que les moyens d'écoulement comprennent au moins une rainure continue (10) pratiquée le long des partie intra-sclérale (1) et partie sous-conjonctivale (5) de façon à déboucher au niveau des extrémité trabéculaire (2) et extrémité externe (6).

3. Implant selon la revendication 1, réalisé en un matériau poreux, les moyens d'écoulement comprenant des passages internes provenant de la porosité du matériau.

4. Implant selon la revendication 1, réalisé en un matériau poreux, caractérisé en ce que les moyens d'écoulement comprennent, d'une part, au moins une rainure continue pratiquée le long des partie intra-sclérale et partie sous-conjonctivale, d'autre part, des passages internes provenant de la porosité du matériau.

5. Implant selon l'une des revendications 2 ou 4, caractérisé en ce que les partie intra-sclérale (1) et partie sous- conjonctivale (5) possèdent des faces supérieures (3, 8) situées sensiblement dans le même plan, et des faces inférieures opposées (4, 9) dotées d'une ou des rainures (10) précitées, décalées de sorte que la face (9) de la partie sous-conjonctivale se trouve en retrait par rapport à celle (4) de la partie intra-sclérale.

6. Implant selon la revendication 5, caractérisé en ce que, à ± 20 % près, l'épaisseur (e₂) de la partie sous-conjonctivale est égale à la moitié de l'épaisseur (e₁) de sa partie intra-sclérale.

7. Implant selon l'une des revendications 1 à 6, caractérisé en ce que l'extrémité trabéculaire (2') présente une forme concave de rayon de courbure sensiblement compris entre 4 mm et 8 mm.

8. Implant selon l'une des revendications 1 à 7, caractérisé en ce que l'extrémité trabéculaire (2) présente une largeur sensiblement comprise entre 2 mm et 4 mm.

9. Implant selon l'une des revendications 1 à 8, caractérisé en ce que les moyens d'arrêt comprennent des épaulements (7a, 7b), situés de part et d'autre de la partie intra-sclérale (1) et définis par une largeur de ladite partie intra-sclérale plus grande que celle de la partie sous-conjonctivale (5).

10. Implant selon la revendication 9, caractérisé en ce que la partie intra-sclérale (1) présente une section frontale sensiblement carrée de côté (c) égal à 3 mm, à ± 20 % près, et la partie sous-conjonctivale (5) une section frontale sensiblement rectangulaire ayant un côté (r₁) égal à 1,5 mm, à ± 20 % près, dans le sens de la largeur de l'implant et un côté (r₂) égal à 2 mm, à ± 20 % près, dans le sens de sa longueur.

11. Implant selon l'une des revendications 1 à 10, réalisé en copolymère méthylmétacrylate/vinylpyrolidone.

## Claims

1. Sclerotomy implant the purpose of which is to enable continuous drainage of aqueous humour across the trabecula of a subject, said implant taking the shape of a platelet made from a biocompatible material and being characterised in that it comprises, in combination:
- a first end, the so-called trabecular end (2), so designed as to be capable of positioning itself against the trabecula,
- a first part, the so-called intra-scleral part (1), comprising the trabecular end (2) and so designed as to be capable of being inserted into a cavity beneath a scleral flap,
- a second part, the so-called subconjunctival part (5), extending the intra-scleral part (1) and having a thickness (e₂) which is less than the thickness (e₁) of said intra-scleral part, said subconjunctival part (5) being so designed as to be capable of projecting from the sclera and of positioning itself beneath the conjunctiva in contact with the latter,
- retaining means (7a, 7b) situated at the junction between the intra-scleral part (1) and the subconjunctival part (5) and so designed as to be capable of ensuring that said intra-scleral part is locked within the cavity,
- a second end, the so-called external end (6), opposite the trabecular end (2) and comprised by the subconjunctival part (5),
- means (10) for continuous drainage extending along the intra-scleral part (1) and the subconjunctival part (5) between the trabecular end (2) and the external end (6) with a view to enabling drainage of the aqueous humour from the trabecula towards the conjunctiva.

2. Implant according to Claim 1, characterised in that the drainage means comprise at least one continuous groove (10) provided along the intra-scleral part (1) and the subconjunctival part (5) in such a manner as to open out at the level of the trabecular end (2) and at the level of the external end (6).

3. Implant according to Claim 1 made from a porous material, with the drainage means comprising internal channels resulting from the porosity of the material.

4. Implant according to Claim 1 made from a porous material, characterised in that the drainage means comprise, on the one hand, at least one continuous groove provided along the intra-scleral part and the subconjunctival part and, on the other hand, internal channels resulting from the porosity of the material.

5. Implant according to one of Claims 2 or 4, characterised in that the intra-scleral part (1) and the subconjunctival part (5) comprise upper faces (3, 8) substantially situated within the same plane and opposite lower faces (4, 9) provided with one or more of the aforementioned grooves (10), said lower faces being offset in such a manner that the face (9) of the subconjunctival part is set back in respect of the face (4) of the intra-scleral part.

6. Implant according to Claim 5, characterised in that the thickness (e₂) of the subconjunctival part is equal, within about ± 20 %, to half the thickness (e₁) of its intra-scleral part.

7. Implant according to one of Claims 1 to 6, characterised in that the trabecular end (2') is concave in shape, with a radius of curvature sustantially between 4 mm and 8 mm.

8. Implant according to one of Claims 1 to 7, characterised in that the trabecular end (2) has a width substantially between 2 mm and 4 mm.

9. Implant according to one of Claims 1 to 8, characterised in that the retaining means comprise shoulders (7a, 7b) situated on either side of the intra-scleral part (1) and defined by a width of said intra-scleral part larger than that of the subconjunctival part (5).

10. Implant according to Claim 9, characterised in that the intra-scleral part (1) has a substantially square front section with a side (c) equal, within about ± 20 %, to 3 mm, and the subconjunctival part (5) has a substantially rectangular front section with, in the direction of the width of the implant, a side (r₁) equal, within about ± 20 %, to 1.5 mm and in the direction of its length a side (r₂) equal, within about ± 20 %, to 2 mm.

11. Implant according to one of Claims 1 to 10, made from methylmetacrylate/vinylpyrolidone copolymer.

## Patentansprüche

1. Sklerotomie-Implantat mit der Aufgabe, kontinuierlichen Abfluß von Augenkammerwasser quer von der einen zu der anderen Seite des Trabekels eines Patienten zu ermöglichen, wobei das besagte Implantat als ein Plättchen aus einem biokompatiblen Material ausgebildet und dadurch gekennzeichnet ist, daß es in Verbindung miteinander die folgenden Teile umfaßt:
- ein erstes Ende, das sogenannte trabekuläre Ende (2), das so beschaffen ist, daß es sich an den Trabekel anlegen kann,
- einen ersten Teil, den sogenannten intraskleralen Teil (1), der das trabekuläre Ende (2) umfaßt und so beschaffen ist, daß er in einen unterhalb einer Skleralklappe befindlichen Hohlraum eingeführt werden kann,
- einen zweiten Teil, den sogenannten subkonjunktivalen Teil (5), der den intraskleralen Teil (1) verlängert und eine Dicke (e₂) aufweist, die geringer ist als die Dicke (e₁) des besagten intraskleralen Teils, wobei der besagte subkonjunktivale Teil (5) so beschaffen ist, daß er aus der Sklera herausragen und sich unterhalb der Bindehaut in Kontakt mit dieser anlegen kann,
- Anschlagmittel (7a, 7b) am Anschluß zwischen dem intraskleralen Teil (1) und dem subkonjunktivalen Teil (5), die so beschaffen sind, daß sie Festlegung des besagten intraskleralen Teils in dem Hohlraum gewährleisten können,
- ein zweites Ende, das sogenannte äußere Ende (6), das dem trabekulären Ende (2) gegenüber liegt und durch den subkonjunktivalen Teil (5) umfaßt wird,
- Mittel (10) für dauernden Abfluß, die sich entlang dem intraskleralen Teil (1) und dem subkonjunktivalen Teil (5) zwischen dem trabekulären Ende (2) und dem äußeren Ende (6) erstrecken und deren Funktion darin besteht, Abfluß des Augenkammerwassers von dem Trabekel auf die Bindehaut zu zu ermöglichen.

2. Implantat nach Anspruch 1, dadurch gekennzeichnet, daß die Abflußmittel mindestens eine kontinuierliche Rille (10) umfassen, die entlang dem intraskleralen Teil (1) und dem subkonjunktivalen Teil (5) so vorgesehen ist, daß sie im Bereiche des trabekulären Endes (2) und im Bereiche des äußeren Endes (6) offen ist.

3. Implantat nach Anspruch 1, das aus einem porösen Material gefertigt ist, wobei die Abflußmittel durch die Porosität des Materials bedingte Innenkanäle umfassen.

4. Implantat nach Anspruch 1, das aus einem porösen Material gefertigt ist, dadurch gekennzeichnet, daß die Abflußmittel einerseits mindestens eine kontinuierliche Rille, die entlang dem intraskleralen Teil und dem subkonjunktivalen Teil vorgesehen ist, und andererseits durch die Porosität des Materials bedingte Innenkanäle umfassen.

5. Implantat nach einem der Ansprüche 2 oder 4, dadurch gekennzeichnet, daß der intrasklerale Teil (1) und der subkonjunktivale Teil (5) obere Flächen (3, 8) aufweisen, die sich im wesentlichen in derselben Ebene befinden, und gegenüberliegende untere Flächen (4, 9), die mit einer oder mehreren der vorstehend genannten Rillen (10) versehen sind, wobei die besagten unteren Flächen so versetzt sind, daß die Fläche (9) des subkonjunktivalen Teils im Verhältnis zu der Fläche (4) des intraskleralen Teils zurückgesetzt ist.

6. Implantat nach Anspruch 5, dadurch gekennzeichnet, daß die Dicke (e₂) des subkonjunktivalen Teils innerhalb von etwa ± 20 % der Hälfte der Dicke (e₁) des intraskleralen Teils des Implantats entspricht.

7. Implantat nach einem der Ansprüche 1 bis 6, dadurch gekennzeichnet, daß das trabekuläre Ende (2') eine konkave Form aufweist, deren Krümmungshalbmesser im wesentlichen zwischen 4 mm und 8 mm beträgt.

8. Implantat nach einem der Ansprüche 1 bis 7, dadurch gekennzeichnet, daß die Breite des trabekulären Endes (2) im wesentlichen zwischen 2 mm und 4 mm beträgt.

9. Implantat nach einem der Ansprüche 1 bis 8, dadurch gekennzeichnet, daß die Haltemittel Schultern (7a, 7b) umfassen, die auf der einen und auf der anderen Seite des intraskleralen Teils (1) angeordnet und durch eine Breite des besagten intraskleralen Teils, die größer ist als die Breite des subkonjunktivalen Teils (5), abgegrenzt sind.

10. Implantat nach Anspruch 9, dadurch gekennzeichnet, daß der intrasklerale Teil (1) ein im wesentlichen quadratisches vorderes Schnittprofil aufweist, wobei die Seite (c) des besagten Schnittprofils innerhalb von etwa ± 20 % 3 mm beträgt, während der subkonjunktivale Teil (5) ein im wesentlichen rechteckiges frontales Schnittprofil aufweist, wobei die Seite (r₁) in Richtung der Implantatbreite innerhalb von etwa ± 20 % 1,5 mm entspricht und die Seite (r₂) in Richtung der Schnittprofillänge innerhalb von etwa ± 20 % 2 mm entspricht.

11. Implantat nach einem der Ansprüche 1 bis 10, das aus einem Mischpolymerisat von Methylmetacrylat und Vinylpyrrolidon besteht.
